# EUROPÄISCHE PATENTSCHRIFT

(11) **EP 2 929 843 B1**
(45) Veröffentlichungstag und Bekanntmachung des Hinweises auf die Patenterteilung: **30.05.2018**
(21) Anmeldenummer: 14164160.5
(22) Anmeldetag: 10.04.2014
(51) Int. Cl.: A61B 17/122

(54) **CHIRURGISCHER CLIP MIT DREI KLEMMARMEN**
SURGICAL CLIP WITH THREE CLAMPING ARMS
CLIP CHIRURGICAL COMPRENANT TROIS BRAS DE SERRAGE

(43) Veröffentlichungstag der Anmeldung: 14.10.2015
(73) Patentinhaber: Lazic Besitz GmbH & Co. KG, 78532 Tuttlingen (DE)
(72) Erfinder: LAZIC, Daniel, 78532 Tuttlingen (DE)
(74) Vertreter: Kohler Schmid Möbus Patentanwälte

(56) Entgegenhaltungen:
- DE-A1-102009 003 273
- DE-U1- 20 303 496

## Beschreibung

Die Erfindung betrifft einen chirurgischen Clip mit Klemmarmen, der beispielsweise zum Abklemmen von Hohlorganen, insbesondere von Blutgefäßen, oder zum Verbinden zweier Blutgefäße dient.

Aus der DE 10 2004 016 859 A1 und der DE 10 2009 003 273 A1 sind Aneurysma-Clips mit zwei drehbar gelagerten Clipteilen bekannt, die jeweils einen Bedienarm und einen Klemmarm aufweisen. Eine Schrauben- bzw. Schenkelfeder ist in einer zentralen Öffnung der beiden Clipteile angeordnet und mit ihren Federschenkeln an den beiden Clipteilen angeschweißt, um die beiden Clipteile in eine geschlossene Clipstellung vorzuspannen, in der die beiden Klemmarme aneinander anliegen.

Aus der DE 203 03 496 U1 ist weiterhin ein chirugischer Clip mit einem ersten Klemmarm und mit einem relativ zum ersten Klemmarm verschwenkbar gelagerten zweiten Klemmarm bekannt. Der erste Klemmarm liegt in einer Klemmstellung mit einem ersten Klemmabschnitt an einem zweiten Klemmabschnitt an, wobei der erste Klemmabschnitt und der zweite Klemmabschnitt aus einem röntgentransparenten Material hergestellt sind.

Es ist die Aufgabe der vorliegenden Erfindung, einen vielseitig einsetzbaren chirurgischen Clip bereitzustellen, mit insbesondere auch zwei Gefäße miteinander verbunden werden können.

Diese Aufgabe wird erfindungsgemäß gelöst durch einen chirurgischen Clip mit drei um eine Drehachse drehbar gelagerten, doppelarmigen Clipteilen, die jeweils einen Bedienarm und einen Klemmarm aufweisen, wobei ein mittleres der Clipteile mit seinem Bedienarm zwischen den Bedienarmen der beiden äußeren Clipteile und mit seinem Klemmarm zwischen den Klemmarmen der beiden äußeren Clipteile angeordnet ist, und mit einer Feder, welche die beiden äußeren Clipteile in eine geschlossene Clipstellung vorspannt, in der die Klemmarme der drei Clipteile aneinander anliegen.

Erfindungsgemäß greift die Feder nur an den beiden äußeren Clipteilen an, so dass das mittlere Clipelement zwischen den beiden äußeren Clipteilen frei drehbar gelagert ist. Mithilfe einer Anlegezange, welche den mittleren Bedienarm und einen der beiden äußeren Bedienarme aufspreizt, können der mittlere Klemmarm und wahlweise einer der beiden äußeren Klemmarme gegeneinander geöffnet werden, wobei der mittlere Klemmarm den außen anliegenden anderen äußeren Klemmarm nach außen mitnimmt, und zwar gegen die Schließkraft der zwischen den beiden äußeren Clipteilen wirkenden Feder. Während der mittlere Klemmarm und der eine äußere Klemmarm geöffnet werden, bleibt der andere äußere Klemmarm in Anlage an dem mittleren Klemmarm gehalten, d.h., der mittlere Klemmarm und der andere äußere Klemmarm bleiben geschlossen. Dadurch kann ein erstes Gefäß zwischen dem mittleren Klemmarm und dem einen äußeren Klemmarm festgeklemmt werden und, ohne diese Klemmung des ersten Gefäßes wieder lösen zu müssen, anschließend ein zweites Gefäß zwischen dem mittleren Klemmarm und dem anderen äußeren Klemmarm festgeklemmt werden. Im Ergebnis sind gleichzeitig zwei Gefäße am Clip festgeklemmt.

In einer bevorzugten Ausführungsform der Erfindung weist eines der drei Clipteile zwischen seinen Klemm- und Bedienarmen eine einstückig angeformte oder unverdrehbar befestigte Lagerwelle oder -hülse auf, wohingegen die beiden anderen Clipteile zwischen ihren Klemm- und Bedienarmen jeweils Lageröffnungen aufweisen, mit denen sie auf der Lagerwelle/Lagerhülse um die Drehachse drehbar gelagert sind. Die Lagerwelle/Lagerhülse bildet somit die Drehlagerung, um die sich die beiden anderen Clipteile drehen. In dieser Ausführungsform besteht der erfindungsgemäße Clip also aus vier Einzelteilen, nämlich den drei Clipteilen und der Feder.

In einer anderen bevorzugten Ausführungsform der Erfindung weisen die drei Clipteile zwischen ihren Klemm- und Bedienarmen jeweils Lageröffnungen auf, mit denen die Clipteile auf einer separaten Lagerwelle oder -hülse um die Drehachse drehbar gelagert sind. Die Lagerwelle/Lagerhülse ist durch die Lageröffnungen der drei Clipteile gesteckt und bildet somit die Drehlagerung, um die sich die drei Clipteile drehen. In dieser Ausführungsform besteht der erfindungsgemäße Clip also aus fünf Einzelteilen, nämlich den drei Clipteilen, der Lagerwelle/Lagerhülse und der Feder. Alternativ kann statt einer separaten Lagerwelle/Lagerhülse auch die Feder die Drehlagerung übernehmen, bei der sich die drei Clipteile mit ihren Lageröffnungen auf der der Feder drehen. In dieser Ausführungsform besteht der Clip also aus vier Einzelteilen, nämlich den drei Clipteilen und der Feder.

Vorzugsweise ist die Feder mit ihren beiden Federenden mit den beiden äußeren Clipteilen stoffschlüssig verbunden, insbesondere verschweißt. Alternativ können die Federschenkel die äußeren Clipteile - ähnlich der Schenkelfeder bei einer Wäscheklammer - lediglich außenseitig umklammern.

Besonders bevorzugt ist die Feder als Schenkelfeder ausgebildet, die mit ihren beiden Federschenkeln an den beiden äußeren Clipteilen angreift. Im Fall einer Lagerhülse sind möglichst alle Schraubenwindungen, also der gesamte Federwindungskörper der Schenkelfeder, vollständig innerhalb der Lagerhülse angeordnet, um Quetschungen von Gewebe zwischen den einzelnen Schraubenwindungen der Schenkelfeder auszuschließen.

Der Verbund aus den drei drehbar gelagerten Clipteilen kann im Fall einer anschweißten Feder prinzipiell durch diese angeschweißte Feder zusammengehalten werden. Zusätzlich oder wenn die Feder nicht anschweißt ist, weist vorzugsweise eines der drei Clipteile zwischen seinen Klemm- und Bedienarmen eine Steckaufnahme auf, in der die anderen zwei Clipteile in einer Montagedrehposition axial eingesteckt und durch anschließendes Verdrehen in Richtung auf die geschlossene Clipstellung miteinander axial verriegelt sind. Dieser Steck-Drehverschluss ermöglicht das Ineinandersetzen der Clipteile und deren Drehführung ohne zusätzliche Bauteile und ohne zusätzlichen Montageaufwand. Im Fall einer separaten Lagerhülse weist die Lagerhülse einenends einen Ringbund und anderenends einen nach außen umgebogenen Nietkopf auf, so dass die drei Clipteile zwischen Ringbund und Nietkopf axial zusammengehalten sind.

Die Klemmarme sind entweder einteilig mit ihren Klemmarmenden ausgebildet oder jeweils zweiteilig mit einer Klemmarmbasis und mit einem Klemmarmende ausgebildet sind, wobei die Klemmarmbasis eine Schnittstelle zum Befestigen des Klemmarmendes aufweist.

In einer besonders bevorzugten Ausführungsform des erfindungsgemäßen Clips sind die Klemmarmenden jeweils als Ringe ausgebildet, die in der geschlossenen Clipstellung aneinander anliegen und mit ihren, insbesondere gleich großen Ringöffnungen eine Durchgangsöffnung ausbilden. Besonders ist dieser Clip für Bypassoperationen geeignet, bei denen zwei Blutgefäße miteinander verbunden werden. Normalerweise müssen die beiden Gefäße zusammengenäht werden, was mit einem großen Zeitaufwand verbunden ist und sehr viel Präzision des Operateurs erfordert. Die beiden offenen Gefäßenden werden jeweils über die Ringe der äußeren Clipteile umgestülpt und durch den Ring des mittlere Clipteils festgeklemmt, wodurch die beiden Gefäßenden ohne Nähen miteinander verbunden sind.

In einer anderen bevorzugten Ausführungsform des erfindungsgemäßen Clips sind die Klemmarme der beiden äußeren Clipteile jeweils als Gabel und der Klemmarm des mittleren Clipteils als Ring ausgebildet sind, wodurch zwei Gefäßwände miteinander verbunden werden können.

In einer weiteren bevorzugten Ausführungsform des erfindungsgemäßen Clips sind der Klemmarm einer der beiden äußeren Clipteile als Gabel und die Klemmarme der beiden anderen Clipteile jeweils als Ring ausgebildet, wodurch ein Gefäßende und eine Gefäßwand miteinander verbunden werden können.

Erfindungsgemäß kann die Verbindung zweier Gefäße, insbesondere bei Bypass-Operationen, deutlich vereinfacht werden. Nach dem Befestigen des ersten Gefäßes kann anschließend das zweite Gefäß befestigt werden, ohne dabei die Verbindung des ersten Gefäßes wieder lösen zu müssen. Die beiden offenen Gefäßenden sind jeweils nach dem Umstülpen über die Ringe der äußeren Clipteile durch den Ring des mittleren Clipteils festgeklemmt, wodurch die beiden Gefäßenden ohne Nähen miteinander verbunden sind.

Weitere Vorteile der Erfindung ergeben sich aus der Beschreibung, den Ansprüchen und der Zeichnung. Ebenso können die vorstehend genannten und die noch weiter aufgeführten Merkmale je für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

Es zeigen:
- Fign. 1a-1c: einen erfindungsgemäßen chirurgischen Clip mit drei drehbar gelagerten doppelarmigen Clipteilen in einer geschlossenen Clipstellung (Fig. 1a), in einer ersten geöffneten Clipstellung (Fig. 1b) und in einer zweiten geöffneten Clipstellung (Fig. 1c);
- Fign. 2a, 2b: das eine äußere Clipteil des in Fig. 1 gezeigten Clips in einer Seitenansicht (Fig. 2a) und in einer axialen Draufsicht (Fig. 2b);
- Fign. 3a, 3b: das mittlere Clipteil des in Fig. 1 gezeigten Clips in einer Seitenansicht (Fig. 3a) und in einer axialen Draufsicht (Fig. 3b);
- Fign. 4a, 4b: das andere äußere Clipteil des in Fig. 1 gezeigten Clips in einer Seitenansicht (Fig. 4a) und in einer axialen Draufsicht (Fig. 4b);
- Fig. 5: die in Fign. 2 bis 4 gezeigten drei Clipteile, die miteinander durch einen Steck-Drehverschluss verbunden sind;
- Fign. 6 bis 8: eine zweite Ausführungsform der drei Clipteile jeweils in einer axialen Draufsicht;
- Fig. 9: die in Fign. 6 bis 8 gezeigten drei Clipteile, die auf einer separaten Lagerhülse drehbar gelagert sind;
- Fig. 10: einen erfindungsgemäßen chirurgischen Clip mit zweiteilig ausgebildeten Klemmarmen;
- Fig. 11: einen erfindungsgemäßen chirurgischen Clip mit ringförmigen Klemmarmen;
- Fign. 12a-12d: die einzelnen Verfahrensschritte beim Verbinden zweier Gefäße mittels des in Fig. 11 gezeigten chirurgischen Clips;
- Fign. 13a,13b: einen weiteren erfindungsgemäßen chirurgischen Clip mit einem ringförmigen, mittleren Klemmarm und zwei doppelnadelförmigen, äußeren Klemmarmen in einer Seitenansicht (Fig. 13a) und in einer axialen Draufsicht (Fig. 13b); und
- Fign. 14a-14d: die einzelnen Verfahrensschritte beim Verbinden zweier Gefäße mittels des in Fig. 13 gezeigten chirurgischen Clips.

In der folgenden Beschreibung der Zeichnung werden für gleiche bzw. funktionsgleiche Bauteile identische Bezugszeichen verwendet.

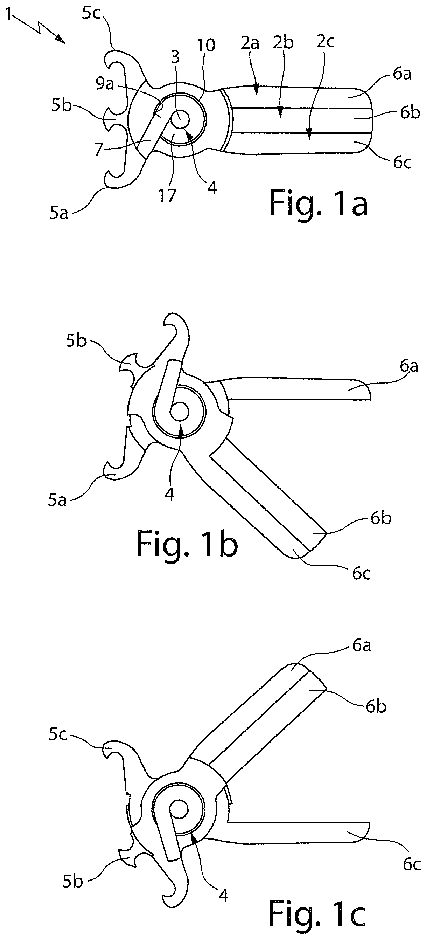

Der in **Fig. 1a** gezeigte chirurgische Clip **1** umfasst drei doppelarmige Clipteile **2a, 2b, 2c,** die um eine Drehachse **3** drehbar miteinander verbunden und durch eine Schenkelfeder **4** in ihre in Fig. 1a gezeigte geschlossene Clipposition vorgespannt sind.

Die doppelarmigen Clipteile 2a, 2b, 2c weisen jeweils einen kurzen Bedienarm **5a, 5b, 5c** und einen langen Klemmarm **6a, 6b, 6c** auf. Das mittlere Clipteil 2b ist mit seinem Bedienarm 5b zwischen den Bedienarmen 5a, 5c der beiden äußeren Clipteile 2a, 2c und mit seinem Klemmarm 6b zwischen den Klemmarmen 6a, 6c der beiden äußeren Clipteile 2a, 2c angeordnet. Die Bedien- und Klemmarme 5a, 6a bzw. 5c, 6c jedes äußeren Clipteils 2a, 2c liegen einander bezüglich der Drehachse 3 gegenüber und sind zueinander parallelversetzt, so dass sich die beiden äußeren Clipteile 2a, 2c bezüglich der Drehachse 3 überkreuzen. Die Schenkelfeder 4 ist mit ihren beiden Federschenkeln 7 an die beiden äußeren Klemmarme 6a, 6c angeschweißt und spannt so die beiden äußeren Clipteile 2a, 2c in die geschlossene Clipstellung vor, in der die Klemmarme 6a, 6b, 6c aneinander anliegen.

Mithilfe einer Anlegezange (nicht gezeigt), welche jeweils zwischen den mittleren Bedienarm 5b und einen der beiden äußeren Bedienarme 5a oder 5c greift und aufspreizt, können der mittlere Klemmarm 6b und der zugehörige äußere Klemmarm 6a oder 6c gegeneinander jeweils gegen die Schließkraft der Schenkelfeder 4 geöffnet werden. Genauer gesagt nimmt beim Öffnen der mittlere Klemmarm 6b in seiner Öffnungsrichtung den anliegenden anderen äußeren Klemmarm 6c bzw. 6a mit, und zwar gegen die auf den anderen äußeren Klemmarm 6c bzw. 6a wirkende Schließkraft der Schenkelfeder 4.

In **Fig. 1b** sind der mittlere Bedienarm 5b und der untere äußere Bedienarme 5a aufgespreizt und dadurch der mittlere Klemmarm 6b und der obere äußere Klemmarm 6a gegeneinander geöffnet. Dabei ist der am mittleren Klemmarm 6b anliegende untere äußere Klemmarm 6c durch den mittleren Klemmarm 6b gegen die Schließkraft der Schenkelfeder 4 in Öffnungsrichtung mitgenommen. In **Fig. 1c** sind der mittlere Bedienarm 5b und der obere äußere Bedienarme 5c aufgespreizt und dadurch der mittlere Klemmarm 6b und der untere äußere Klemmarm 6c gegeneinander geöffnet. Dabei ist der am mittleren Klemmarm 6b anliegende obere äußere Klemmarm 6a durch den mittleren Klemmarm 6b gegen die Schließkraft der Schenkelfeder 4 in Öffnungsrichtung mitgenommen.

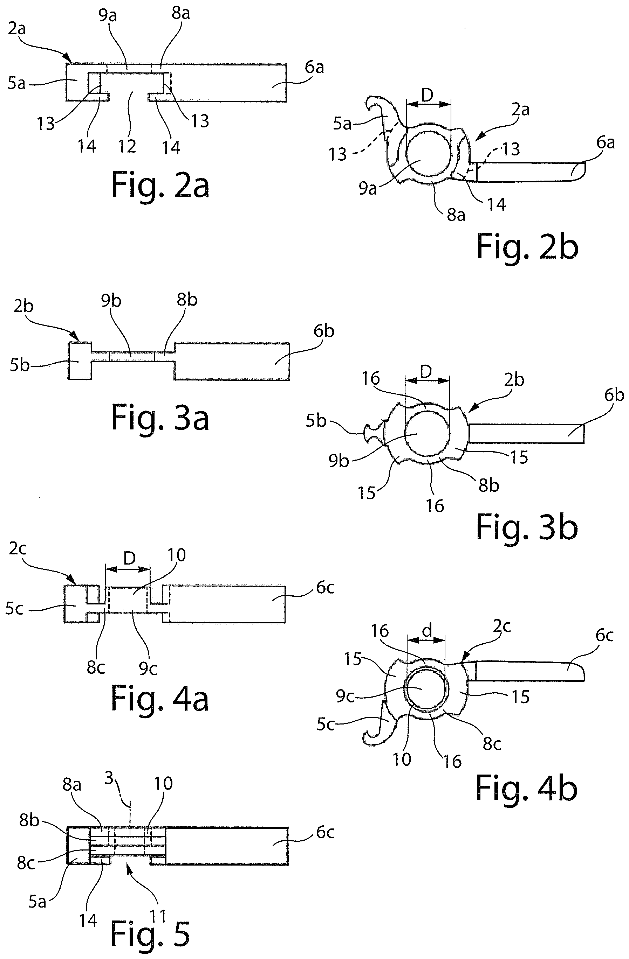

Wie in **Fign. 2a, 2b** gezeigt ist, weist das eine äußere Clipteil 2a zwischen Bedien- und Klemmarm 5a, 6a einen flachen Ringabschnitt **8a** mit einer kreisrunden Lageröffnung **9a** (Öffnungsdurchmesser **D**) auf. Wie in **Fign. 3a, 3b** gezeigt ist, weist das mittlere Clipteil 2b zwischen Bedien- und Klemmarm 5b, 6b ebenfalls einen flachen Ringabschnitt **8b** mit einer kreisrunden Lageröffnung **9b** (Öffnungsdurchmesser D) auf. Wie in **Fign. 4a, 4b** gezeigt ist, weist das andere äußere Clipteil 2c zwischen Bedien- und Klemmarm 5c, 6c einen flachen Ringabschnitt **8c** mit einer angeformten axialen Lagerhülse **10** (Außendurchmesser D, Innendurchmesser d) auf, deren Hülsenöffnung mit **9c** bezeichnet ist.

Wie in **Fig. 5** gezeigt, sind die beiden Clipteile 2a, 2b mit ihren Lageröffnungen 9a, 9b auf die Lagerhülse 10 des Clipteils 2c aufgesteckt und dadurch gegeneinander um die Achse 3 der Lagerhülse 10 drehbar gelagert. Außerdem sind die drei Clipteile 2a, 2b, 2c miteinander durch einen Steck-Drehverschluss **11** axial verbunden. Das äußere Clipteil 2a weist für die Ringabschnitte 8b, 8c der beiden anderen Clipteile 2b, 2c eine axial offene Steckaufnahme **12** auf, deren Boden durch den Ringabschnitt 9a gebildet ist. Die Steckaufnahme 12 ist durch zwei bezüglich der Lageröffnung 9a einander gegenüberliegende Seitenwände **13** gebildet, die jeweils mit einem Vorsprung **14** den Ringabschnitt 9a in einem Abstand übergreifen, welcher der Dicke der beiden Ringabschnitte 8b, 8b entspricht. Die beiden Ringabschnitte 8b, 8c der beiden anderen Clipteile 2b, 2c weisen jeweils zwei bezüglich der Lager- bzw. Hülsenöffnung 9b, 9c einander gegenüberliegende erste Ringsegmente **15** und dazwischen jeweils ein zweites Ringsegment **16** auf, wobei die zweiten Ringsegmente 16 gegenüber den ersten Ringsegmenten 15 radial nach innen zurückversetzt sind.

Zum Montieren des Clips 1 werden die beiden Clipteile 2b, 2c mit ihren zweiten Ringsegmenten 16 zwischen den beiden Vorsprüngen 14 des Clipteils 2a ausgerichtet und in dieser maximal weit geöffneten Montagedrehposition axial ineinander gesteckt, wobei gleichzeitig die Clipteile 2b, 2c auf die Lagerhülse 10 des Clipteils 2c aufgesteckt werden. Anschließend werden für den Drehsteckverschluss die beiden Clipteile 2b, 2c in Richtung auf die geschlossene Clipstellung gedreht, wodurch die ersten Ringsegmente 15 der Clipteils 2b, 2c zwischen dem Ringabschnitt 8a und den Vorsprüngen 14 miteinander axial verbunden bzw. entgegen der Steckrichtung verriegelt sind. Abschließend wird die Schenkelfeder 4 mit ihrem Federwindungskörper **17** (Fig. 1a) in die Lagerhülse 10 eingefädelt und dann mit ihren beiden Federschenkeln 7 außenseitig an den Klemmarmen 6a, 6c unter Vorspannung angeschweißt, um die Clipteile 2a, 2c bereits in ihrer geschlossenen Clipstellung vorzuspannen. In dieser Ausführungsform besteht der Clip 1 also aus vier Einzelteilen, nämlich den drei Clipteilen 2a, 2b, 2c und der Feder 4.

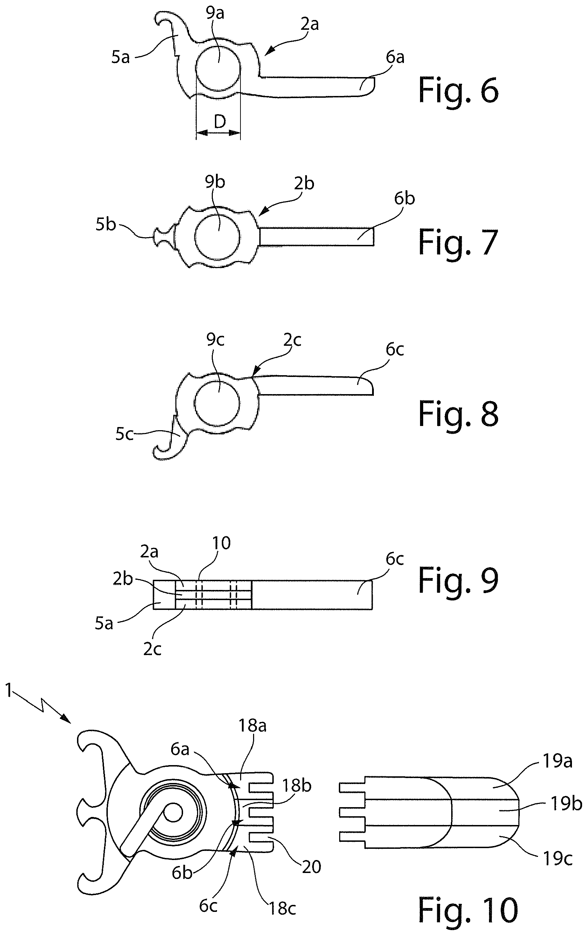

In **Fign. 6 bis 8** sind die drei Clipteile 2a, 2b, 2c eines modifizierten Clips gezeigt, bei dem die Lagerhülse 10 nicht Teil eines Clipteils ist, sondern ein separates Teil ist und alle drei Clipteile 2a, 2b, 2c jeweils die gleiche Lageöffnung 9a, 9b, 9c mit Durchmesser D aufweisen. Durch diese Lageröffnungen 9a, 9b, 9c wird dann die Lagerhülse 10 gesteckt (**Fig. 9**), deren runder Außendurchmesser bis auf ein minimales Lagerspiel dem Öffnungsdurchmesser D der kreisrunden Lageröffnungen entspricht. Die Lagerhülse 10 bildet somit die Drehlagerung, um die sich alle drei Clipteile drehen. Abschließend wird die Schenkelfeder 4 mit ihrem Federwindungskörper 17 in die Lagerhülse 10 eingefädelt und dann mit ihren beiden Federschenkeln 7 außenseitig an den Klemmarmen 6a, 6c unter Vorspannung angeschweißt. In dieser Ausführungsform besteht der Clip 1 also aus fünf Einzelteilen, nämlich den drei Clipteilen 2a, 2b, 2c, der Lagerhülse 10 und der Schenkelfeder 4.

Anstelle einer Lagerhülse kann die Drehlagerung auch durch eine massive Lagerwelle gebildet sein, allerdings muss dann der Federwindungskörper 17 außen am Clip 1 angeordnet werden.

Bei einer nicht gezeigten Ausführungsform, die ohne Lagerhülse auskommt, wird der Federwindungskörper 17 der Schenkelfeder 4, dessen runder Außendurchmesser in diesem Fall bis auf ein minimales Lagerspiel dem Öffnungsdurchmesser D der kreisrunden Lageröffnungen entspricht, durch die Lageröffnungen 9a, 9b, 9c gesteckt. Die Schenkelfeder 4 bildet somit die Drehlagerung, um die sich alle drei Clipteile drehen. In dieser Ausführungsform besteht der Clip 1 also aus vier Einzelteilen, nämlich den drei Clipteilen 2a, 2b, 2c und der Schenkelfeder 4.

Statt mit den beiden Clipteilen 2a, 2c verschweißt zu werden, können alternativ die beiden Federschenkel 7 die beiden Klemmarme 6a, 6c - ähnlich einer Wäscheklammer - außenseitig umklammern. In diesem Fall können die beide Clipteile 2a, 2b, 2c auch aus nicht-schweißbarem Material, wie z.B. aus Kunststoff, insbesondere aus Polymethylmethacrylat (PMMA) oder aus röntgentransparentem Polyetheretherketon (PEEK), gebildet sein. Im Falle der separaten Lagerhülse 10 können die Hülsenende beispielsweise radial nach außen umgebogen sein, um die Clipteile unverlierbar auf der Lagerhülse 10 zu befestigen.

Vom Clip 1 der Fig. 1 unterscheidet sich der in **Fig. 10** gezeigte Clip 1 einzig dadurch, dass hier die Klemmarme 6a, 6b, 6c jeweils zweiteilig mit einer Klemmarmbasis **18a, 18b, 18c** und mit einem Klemmarmende **19a, 19b, 19c** ausgebildet sind. Die Klemmarmbasis 18a, 18b, 18c weist eine z.B. als Steckaufnahme ausgebildete Schnittstelle **20** zum wahlweisen Befestigen von unterschiedlichen Klemmarmenden 19a, 19b, 19c auf.

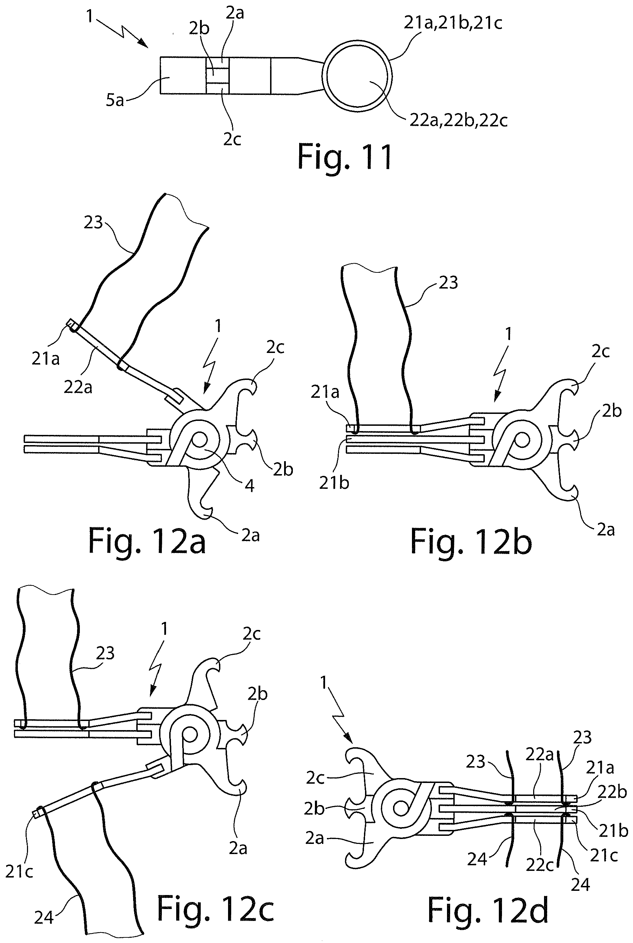

Bei dem in **Fig. 11** gezeigten chirurgischen Clip 1 sind die Klemmarmenden jeweils als Ringe **21a, 21b, 21c** mit gleich großer Ringöffnung **22a, 22b, 22c** ausgebildet sind, die in der geschlossenen Clipstellung aneinander anliegen und sich mit ihren Ringöffnungen 22a, 22b, 22c decken, um so eine Durchgangsöffnung auszubilden.

In Fign. 12a-12d sind die einzelnen Verfahrensschritte beim Verbinden zweier Gefäße (z.B. Blutgefäße) **23, 24** mittels des in Fig. 11 gezeigten chirurgischen Clips 1 gezeigt:
In **Fig. 12a** ist der Clip 1 zwischen dem ersten äußeren Clipteil 2a und dem mittleren Clipteil 2b geöffnet, und das offene Gefäßende des ersten Blutgefäßes 23 ist durch die Ringöffnung 22a des ersten äußeren Clipteils 2a geführt und dann um den Ring 21a des ersten äußeren Clipteils 2a nach außen umgestülpt.

In **Fig. 12b** ist der Clip 1 wieder geschlossen und dadurch das umgestülpte Gefäßende zwischen den Ringen 21a, 21b des ersten äußeren Clipteils 2a und des mittleren Clipteils 2b durch die Schließkraft er Schenkelfeder 4 festgeklemmt. In **Fig. 12c** ist der Clip 1 zwischen dem zweiten äußeren Clipteil 2c und dem mittleren Clipteil 2b geöffnet, und das offene Gefäßende des zweiten Blutgefäßes 24 ist durch die Ringöffnung 22c des zweiten äußeren Clipteils 2c geführt und dann um den Ring 21c des zweiten äußeren Clipteils 2c umgestülpt.
In **Fig. 12d** ist der Clip 1 wieder geschlossen und dadurch das umgestülpte Gefäßende des zweiten Blutgefäßes 24 zwischen den Ringen 21c, 21b des zweiten äußeren Clipteils 2c und des mittleren Clipteils 2b festgeklemmt. Die über die Ringe 21a, 21c der äußeren Clipteile 2a, 2c umgestülpten offenen Gefäßenden der beiden Blutgefäße 23, 24 sind durch den Ring 21b des mittleren Clipteils 2b festgeklemmt und ohne Nähen miteinander verbunden.

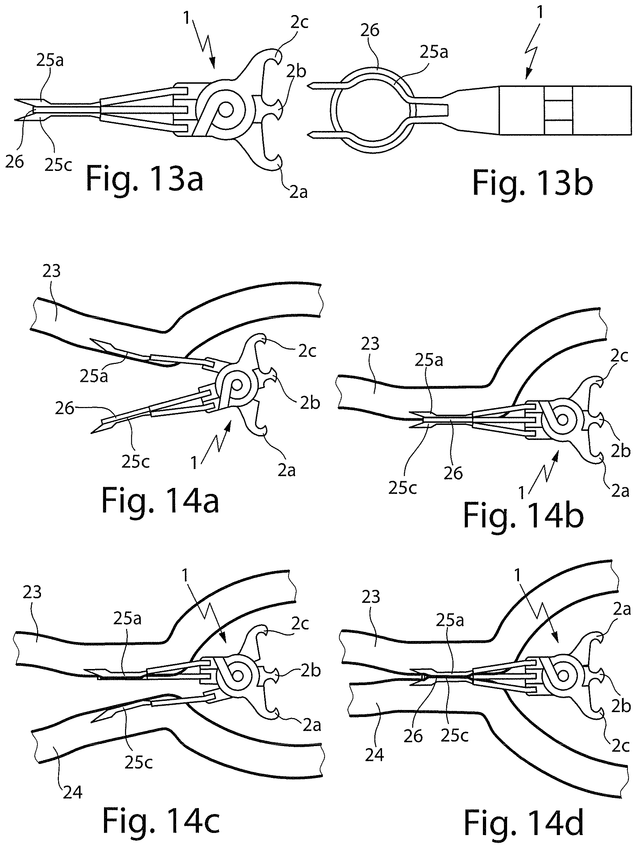

Bei dem in **Fig. 13a, 13b** gezeigten chirurgischen Clip 1 sind die Klemmarmenden der beiden äußeren Clipteile 2a, 2c jeweils als Gabel (Doppelnadel) **25a, 25c** und das Klemmarmende des mittleren Clipteils 2b als Ring **26** ausgebildet. Die Gabeln 25a, 25c weisen jeweils kreisbogenförmig nach außen gebauchte Mittelabschnitte auf, die in der geschlossenen Clipstellung an dem Ring 26 des mittleren Clipteils 2b anliegen.

In Fign. 14a-14d sind die einzelnen Verfahrensschritte beim Verbinden zweier Blutgefäße 23, 24 mittels des in Fig. 13 gezeigten chirurgischen Clips 1 gezeigt:
In **Fig. 14a** ist der Clip 1 zwischen dem ersten äußeren Clipteil 2a und dem mittleren Clipteil 2b geöffnet und mit der Gabel 25a des ersten äußeren Clipteils 2a in die Gefäßwand des ersten Blutgefäßes 23 eingestochen.
In **Fig. 14b** ist der Clip 1 wieder geschlossen und dadurch die eingestochene Gefäßwand zwischen der Gabel 25a des ersten äußeren Clipteils 2a und dem Ring 26 des mittleren Clipteils 2b durch die Schließkraft der Schenkelfeder 4 festgeklemmt.
In **Fig. 14c** ist der Clip 1 zwischen dem zweiten äußeren Clipteil 2a und dem mittleren Clipteil 2b geöffnet und mit der Gabel 25c des zweiten äußeren Clipteils 2c in die Gefäßwand des zweiten Blutgefäßes 24 eingestochen.

In **Fig. 14d** ist der Clip 1 wieder geschlossen, wodurch das eingestochene erste Blutgefäß 23 zwischen der Gabel 25a und dem Ring 26 und das eingestochene zweite Blutgefäß 24 zwischen der Gabel 25c und dem Ring 26 festgeklemmt sind. Abschließend werden die beiden Gefäßwände innerhalb des Ringes 26 geöffnet, wodurch die beiden Blutgefäße 23, 24 miteinander verbunden sind.

Bei einer nicht gezeigten Ausführungsform sind der Klemmarm einer der beiden äußeren Clipteile als Gabel und die Klemmarme der beiden anderen Clipteile jeweils als Ring ausgebildet, um einerseits das Gefäßende eines ersten Blutgefäßes zwischen den beiden Ringen festzuklemmen und andererseits die eingestochene Gefäßwand eines zweiten Blutgefäßes zwischen einem Ring und der Gabel festzuklemmen. Abschließend wird die eingestochene Gefäßwand innerhalb des Ringes geöffnet, wodurch die beiden Blutgefäße 23, 24 miteinander verbunden sind.

## Patentansprüche

1. Chirurgischer Clip (1) mit drei um eine Drehachse (3) drehbar gelagerten, doppelarmigen Clipteilen (2a, 2b, 2c), die jeweils einen Bedienarm (5a, 5b, 5c) und einen Klemmarm (6a, 6b, 6c) aufweisen, wobei ein mittleres (2b) der Clipteile mit seinem Bedienarm (5b) zwischen den Bedienarmen (5a, 5c) der beiden äußeren Clipteile (2a, 2c) und mit seinem Klemmarm (6b) zwischen den Klemmarmen (6a, 6c) der beiden äußeren Clipteile (2a, 2c) angeordnet ist, und mit einer Feder (4), welche die beiden äußeren Clipteile (2a, 2c) in eine geschlossene Clipstellung vorspannt, in der die Klemmarme (6a, 6b, 6c) der drei Clipteile (2a, 2b, 2c) aneinander anliegen.

2. Chirurgischer Clip nach Anspruch 1, **dadurch gekennzeichnet, dass** eines (2c) der drei Clipteile zwischen seinen Klemm- und Bedienarmen (5c, 6c) eine Lagerwelle oder -hülse (10) aufweist und dass die beiden anderen Clipteile (2a, 2b) zwischen ihren Klemm- und Bedienarmen (5a, 6a; 5b 6b) jeweils Lageröffnungen (9a, 9b) aufweisen, mit denen sie auf der Lagerhülse (10) um die Drehachse (3) drehbar gelagert sind.

3. Chirurgischer Clip nach Anspruch 1, **dadurch gekennzeichnet, dass** die drei Clipteile (2a, 2b, 2c) zwischen ihren Klemm- und Bedienarmen (5a, 6a; 5b, 6b; 5c, 6c) jeweils Lageröffnungen (9a, 9b, 9c) aufweisen, mit denen die Clipteile (2a, 2b, 2c) auf einer separaten Lagerwelle oder -hülse (10) um die Drehachse (3) drehbar gelagert sind.

4. Chirurgischer Clip nach einem der vorhergehenden Ansprüche, **dadurch gekennzeichnet, dass** die Feder (4) mit ihren beiden Federenden (7) mit den beiden äußeren Clipteilen (2a, 2c) stoffschlüssig verbunden, insbesondere verschweißt ist.

5. Chirurgischer Clip nach einem der vorhergehenden Ansprüche, **dadurch gekennzeichnet, dass** die Feder als Schenkelfeder (4) ausgebildet ist und mit ihrem Windungskörper (17) zumindest teilweise in einer Lagerhülse (10) aufgenommen ist.

6. Chirurgischer Clip nach einem der vorhergehenden Ansprüche, **dadurch gekennzeichnet, dass** eines (2a) der drei Clipteile zwischen seinen Klemm- und Bedienarmen (5a, 6a) eine Steckaufnahme (12) aufweist, in der die anderen zwei Clipteile (2b, 2c) in einer Montagedrehposition axial eingesteckt und durch anschließendes Verdrehen in Richtung auf die geschlossene Clipstellung miteinander axial verriegelt sind.

7. Chirurgischer Clip nach einem der vorhergehenden Ansprüche, **dadurch gekennzeichnet, dass** die Klemmarme (6a, 6b, 6c) einteilig mit ihren Klemmarmenden ausgebildet sind.

8. Chirurgischer Clip nach einem der Ansprüche 1 bis 6, **dadurch gekennzeichnet, dass** die Klemmarme (6a, 6b, 6c) jeweils zweiteilig mit einer Klemmarmbasis (18a, 18b, 18c) und mit einem Klemmarmende (19a, 19b, 19c) ausgebildet sind und dass die Klemmarmbasis (18a, 18b, 18c) eine Schnittstelle (20) zum Befestigen des Klemmarmendes (19a, 19b, 19c) aufweist.

9. Chirurgischer Clip nach einem der vorhergehenden Ansprüche, **dadurch gekennzeichnet, dass** die Klemmarme jeweils als Ringe (21a, 21b, 21c) ausgebildet sind, die in der geschlossenen Clipstellung aneinander anliegen und mit ihren Ringöffnungen (22a, 22b, 22c) eine Durchgangsöffnung ausbilden.

10. Chirurgischer Clip nach einem der Ansprüche 1 bis 8, **dadurch gekennzeichnet, dass** die Klemmarme der beiden äußeren Clipteile (2a, 2c) jeweils als Gabel (25a, 25c) und der Klemmarm des mittleren Clipteils (2b) als Ring (26) ausgebildet sind.

11. Chirurgischer Clip nach einem der Ansprüche 1 bis 8, **dadurch gekennzeichnet, dass** der Klemmarm einer der beiden äußeren Clipteile (2a, 2c) als Gabel und die Klemmarme der beiden anderen Clipteile jeweils als Ring ausgebildet sind.

## Claims

1. Surgical clip (1) comprising three double-armed clip parts (2a, 2b, 2c) that are mounted to be rotatable about an axis of rotation (3) and each comprise one operating arm (5a, 5b, 5c) and one clamping arm (6a, 6b, 6c), wherein a middle clip part (2b) is arranged such that its operating arm (5b) is arranged between the operating arms (5a, 5c) of the two outer clip parts (2a, 2c) and its clamping arm (6b) is arranged between the clamping arms (6a, 6c) of the two outer clip parts (2a, 2c), and comprising a spring (4) that pretensions the two outer clip parts (2a, 2c) into a closed clip position in which the clamping arms (6a, 6b, 6c) of the three clip parts (2a, 2b, 2c) abut one another.

2. Surgical clip according to claim 1, **characterized in that** one (2c) of the three clip parts comprises a bearing shaft or bearing sleeve (10) between its clamping and operating arms (5c, 6c), and the other two clip parts (2a, 2b) each comprise a bearing opening (9a, 9b) between their clamping and operating arms (5a, 6a; 5b, 6b), by means of which they are disposed on the bearing sleeve (10) such that they can rotate about the axis of rotation (3).

3. Surgical clip according to claim 1, **characterized in that** each of the three clip parts (2a, 2b, 2c) comprises a bearing opening (9a, 9b, 9c) between its clamping and operating arms (5a, 6a; 5b, 6b; 5c, 6c), by means of which bearing openings, the clip parts (2a, 2b, 2c) are mounted on a separate bearing shaft or bearing sleeve (10) such that they can rotate about the axis of rotation (3).

4. Surgical clip according to any one of the preceding claims, **characterized in that** the two spring ends (7) of the spring (4) are connected to the two outer clip parts (2a, 2c) in a material-bonding manner, in particular through welding.

5. Surgical clip according to any one of the preceding claims, **characterized in that** the spring is designed as a leg spring (4), the winding body (17) of which is at least partially accommodated in a bearing sleeve (10).

6. Surgical clip according to any one of the preceding claims, **characterized in that** one (2a) of the three clip parts comprises a receiving area (12) between its clamping and operating arms (5a, 6a), into which the other two clip parts (2b, 2c) are axially inserted in an assembly rotary position and are axially interlocked by subsequent rotation in the direction towards the closed clip position.

7. Surgical clip according to any one of the preceding claims, **characterized in that** the clamping arms (6a, 6b, 6c) are designed in one piece with their clamping arm ends.

8. Surgical clip according to any one of the claims 1 to 6, **characterized in that** the clamping arms (6a, 6b, 6c) are designed in each case in two parts with a clamping arm base (18a, 18b, 18c) and a clamping arm end (19a, 19b, 19c), and the clamping arm base (18a, 18b, 18c) comprises an interface (20) for fixing the clamping arm end (19a, 19b, 19c).

9. Surgical clip according to any one of the preceding claims, **characterized in that** the clamping arms are designed in each case as rings (21a, 21b, 21c) that abut each other in the closed clip position and the ring openings (22a, 22b, 22c) of which form a through-hole.

10. Surgical clip according to any one of the claims 1 to 8, **characterized in that** the clamping arms of the two outer clip parts (2a, 2c) are each designed as a fork (25a, 25c) and the clamping arm of the middle clip part (2b) is designed as a ring (26).

11. Surgical clip according to any one of the claims 1 to 8, **characterized in that** the clamping arm of one of the two outer clip parts (2a, 2c) is designed as a fork and the clamping arms of the other two clip parts are each designed as a ring.

## Revendications

1. Pince chirurgicale (1) constituée de trois parties (2a, 2b, 2c) à bras doubles, montées de manière rotative autour d'un axe de rotation (3) et comprenant, respectivement, un bras d'actionnement (5a, 5b, 5c) et un bras de coincement (6a, 6b, 6c), une partie médiane (2b), parmi lesdites parties de la pince, étant interposée par son bras d'actionnement (5b) entre les bras d'actionnement (5a, 5c) des deux parties extérieures (2a, 2c) de ladite pince et, par son bras de coincement (6b), entre les bras de coincement (6a, 6c) desdites deux parties extérieures (2a, 2c) de la pince ; et d'un ressort (4) qui précontraint les deux parties extérieures (2a, 2c) de la pince vers une position fermée de serrage dans laquelle les bras de coincement (6a, 6b, 6c) des trois parties (2a, 2b, 2c) de ladite pince sont en applique les uns contre les autres.

2. Pince chirurgicale selon la revendication 1, **caractérisée par le fait que** l'une (2c) des trois parties de ladite pince est munie d'un arbre ou d'une douille de montage (10) entre ses bras de coincement et d'actionnement (6c, 5c) ; et **par le fait que** les deux autres parties (2a, 2b) de ladite pince sont respectivement pourvues, entre leurs bras de coincement et d'actionnement (6a, 5a; 6b, 5b), d'orifices de montage (9a, 9b) par lesquels elles sont montées sur ladite douille de montage (10), de manière rotative autour de l'axe de rotation (3).

3. Pince chirurgicale selon la revendication 1, **caractérisée par le fait que** les trois parties (2a, 2b, 2c) de ladite pince sont respectivement dotées, entre leurs bras de coincement et d'actionnement (6a, 5a ; 6b, 5b ; 6c, 5c), d'orifices de montage (9a, 9b, 9c) par lesquels lesdites parties (2a, 2b, 2c) de ladite pince sont montées de manière rotative, autour de l'axe de rotation (3), sur un arbre ou une douille de montage (10) distinct(e).

4. Pince chirurgicale selon l'une des revendications précédentes, **caractérisée par le fait que** le ressort (4) est relié aux deux parties extérieures (2a, 2c) de ladite pince par ses deux extrémités (7), matériellement et notamment par soudage.

5. Pince chirurgicale selon l'une des revendications précédentes, **caractérisée par le fait que** le ressort est réalisé sous la forme d'un ressort (4) à branches et est, au moins partiellement, logé dans une douille de montage (10) par son corps spiralé (17).

6. Pince chirurgicale selon l'une des revendications précédentes, **caractérisée par le fait que** l'une (2a) des trois parties de ladite pince est pourvue, entre ses bras de coincement et d'actionnement (6a, 5a), d'un logement d'emboîtement (12) dans lequel les deux autres parties (2b, 2c) de ladite pince sont emboîtées axialement dans une position de montage prise par rotation, et sont verrouillées axialement l'une à l'autre par rotation consécutive en direction de la position fermée de serrage.

7. Pince chirurgicale selon l'une des revendications précédentes, **caractérisée par le fait que** les bras de coincement (6a, 6b, 6c) forment une seule pièce par leurs extrémités.

8. Pince chirurgicale selon l'une des revendications 1 à 6, **caractérisée par le fait que** les bras de coincement (6a, 6b, 6c) sont réalisés en deux parties comprenant, respectivement, une base (18a, 18b, 18c) et une extrémité (19a, 19b, 19c) ; et **par le fait que** la base (18a, 18b, 18c) desdits bras de coincement comporte une région entaillée (20) dévolue à la fixation de l'extrémité (19a, 19b, 19c) desdits bras de coincement.

9. Pince chirurgicale selon l'une des revendications précédentes, **caractérisée par le fait que** les bras de coincement sont respectivement réalisés sous la forme d'anneaux (21a, 21b, 21c) qui, dans la position fermée de serrage, sont en applique les uns contre les autres et forment une ouverture de passage par leurs orifices annulaires (22a, 22b, 22c).

10. Pince chirurgicale selon l'une des revendications 1 à 8, **caractérisée par le fait que** les bras de coincement des deux parties extérieures (2a, 2c) de ladite pince sont respectivement réalisés sous la forme d'une fourche (25a, 25c), et le bras de coincement de la partie médiane (2b) de ladite pince est réalisé sous la forme d'un anneau (26).

11. Pince chirurgicale selon l'une des revendications 1 à 8, **caractérisée par le fait que** le bras de coincement de l'une des deux parties extérieures (2a, 2c) de ladite pince est réalisé sous la forme d'une fourche, et les bras de coincement des deux autres parties de ladite pince sont respectivement réalisés sous la forme d'un anneau.
